# EUROPÄISCHE PATENTSCHRIFT

(11) **EP 1 375 471 B1**
(45) Veröffentlichungstag und Bekanntmachung des Hinweises auf die Patenterteilung: **02.01.2008**
(21) Anmeldenummer: 03012042.2
(22) Anmeldetag: 28.05.2003
(51) Int. Cl.: C07C 217/90

(54) **Bis-o-amino(thio)phenole und deren Herstellung**
Bis-o-amino(thio)phenols and their preparation
Bis-o-amino(thio)phénols et leur préparation

(30) Priorität: 27.06.2002 DE 10228763
(43) Veröffentlichungstag der Anmeldung: 02.01.2004
(73) Patentinhaber: Infineon Technologies AG, 81669 München (DE)
(72) Erfinder: Walter, Andreas, 91349 Egloffstein (DE); Gnüchtel, Ingo, 86504 Merching (DE); Maltenberger, Anna, 91359 Leutenbach (DE); Sezi, Recai, 91341 Röttenbach (DE); Hartmann, Horst, 06217 Merseburg (DE)
(74) Vertreter: Müller - Hoffmann & Partner

(56) Entgegenhaltungen:
- EP-A- 0 906 903
- EP-A- 1 132 374
- DE-A- 10 136 382

## Beschreibung

Die Erfindung betrifft Bis-o-aminophenole sowie Verfahren zu deren Herstellung. Die Bis-o-aminophenole eignen sich für die Herstellung von Poly-o-hydroxyamiden, welche nach Umsetzung zu den entsprechenden Polybenzoxazolen als Dielektrikum in Mikrochips verwendet werden können.

Um ein durch kapazitive Kopplung verursachtes Übersprechen von Signalen zu vermeiden, werden in Mikrochips benachbarte Leiterbahnen durch ein zwischen den Leiterbahnen angeordnetes Dielektrikum voneinander isoliert. Verbindungen, die als Dielektrikum eingesetzt werden sollen, müssen verschiedene Anforderungen erfüllen. So hängt die Signallaufzeit in Mikrochips sowohl vom Material der Leiterbahn ab, wie auch vom Dielektrikum, das zwischen den Leiterbahnen angeordnet ist. Je geringer die Dielektrizitätskonstante des Dielektrikums ist, um so geringer ist auch die Signallaufzeit. Die bisher verwendeten Dielektrika auf der Basis von Siliziumdioxid besitzen eine Dielektrizitätskonstante von ca. 4. Diese Materialien werden nach und nach durch organische Dielektrika ersetzt, die eine deutlich niedrigere Dielektrizitätskonstante aufweisen. Die Dielektrizitätskonstante dieser Materialien liegt meist unterhalb von 3.

In den gegenwärtig gebräuchlichen Mikrochips bestehen die Leiterbahnen bevorzugt aus Aluminium, AlCu oder AlCuSi. Mit zunehmender Integrationsdichte der Speicherchips geht man wegen seines im Vergleich zu Aluminium geringeren elektrischen Widerstandes auf Kupfer als Leiterbahnmaterial über. Kupfer erlaubt kürzere Signallaufzeiten und damit eine Verringerung des Leiterbahnquerschnittes. Im Gegensatz zu den bisher gebräuchlichen Techniken, bei denen das Dielektrikum in den Gräben zwischen den Leiterbahnen eingefüllt wird, strukturiert man in der Kupfer-Damascene-Technik zuerst das Dielektrikum. Die dabei entstehenden Gräben werden zunächst mit einer sehr dünnen Barriere beschichtet, die beispielsweise aus Titan, Titannitrid, Tantal, Tantalnitrid, Siliziumcarbid, Siliziumnitrid oder Siliziumcarbonitrid besteht. Diese Barriere ist notwendig, um zu vermeiden, dass Metallatome aus der Leiterbahn in das umgebende Dielektrikum diffundieren, wenn während der Herstellung der Mikrochips Produktionsstufen durchlaufen werden, welche Temperaturen von 400°C oder darüber erfordern. Anschließend werden die Gräben zunächst mit Kupfer gefüllt und daraufhin überschüssiges Kupfer mechanisch abgeschliffen. Das Dielektrikum muss daher stabil gegenüber den zum Schleifen verwendeten Materialien sein und eine ausreichende Haftung auf dem Untergrund aufweisen, um während des mechanischen Schleifprozesses nicht abgelöst zu werden. Ferner muss das Dielektrikum in den folgenden Prozessschritten eine ausreichende Stabilität aufweisen, in welchen weitere Bauelemente des Mikrochips erzeugt werden. Dazu muss es beispielsweise eine ausreichende Temperaturstabilität aufweisen und auch bei Temperaturen von mehr als 400°C keine Zersetzung erleiden. Ferner muss das Dielektrikum gegenüber Prozesschemikalien, wie Lösungsmitteln, Strippern, Basen, Säuren oder aggressiven Gasen stabil sein. Weitere Anforderungen sind eine gute Löslichkeit und ausreichende Lagerstabilität der Vorstufen, aus welchen die Dielektrika erzeugt werden.

Polybenzoxazole (PBO) sind Polymere, die eine sehr hohe Wärmebeständigkeit aufweisen. Die Substanzen werden bereits zur Herstellung von Schutz- und Isolierschichten verwendet. Polybenzoxazole können durch Cyclisierung aus Poly-o-hydroxyamiden hergestellt werden. Die Poly-o-hydroxyamide zeigen eine gute Löslichkeit in organischen Lösungsmitteln, sowie gute Filmbildungseigenschaften. Sie lassen sich mittels Schleudertechnik einfach auf elektronische Bauteile aufbringen. Nach einer Temperaturbehandlung, bei welcher das Poly-o-hydroxyamid zum Polybenzoxazol cyclisiert wird, enthält man ein Polymer, das die gewünschten Eigenschaften aufweist. Polybenzoxazole lassen sich auch direkt in ihrer cyclisierten Form verarbeiten. In der Regel bestehen jedoch in diesem Fall Schwierigkeiten mit der Löslichkeit des Polymers. Der bei der Cyclisierung von Poly-o-hydroxyamiden zu Polybenzoxazolen ablaufende Mechanismus ist im Folgenden schematisch dargestellt:

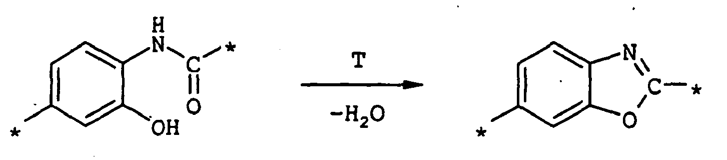

Beim Erhitzen cyclisiert das o-Hydroxyamid zum Oxazol, wobei Wasser freigesetzt wird.

Die Poly-o-hydroxyamide werden durch Umsetzung von Bis-o-aminophenolen mit Dicarbonsäuren hergestellt. Die Eigenschaften des Poly-o-aminophenols und des daraus hergestellten Polybenzoxazols werden wesentlich von den als Ausgangsprodukt verwendeten Monomeren bestimmt. So werden nicht nur das thermische, elektrische oder mechanische Verhalten, sondern auch die Löslichkeit, Hydrolysestabilität, Lagerfähigkeit und zahlreiche weitere Eigenschaften der Polymere durch die Art des bei der Herstellung verwendeten Aminophenols beeinflusst. Um Polybenzoxazole zur Verfügung stellen zu können, die in der Mikroelektronik als Dielektrikum zwischen zwei Metallebenen, zum Beispiel bei Multi-Chip-Modulen, Speicher- und Logikchips oder als Pufferschicht zwischen dem Chip und seinem Gehäuse verwendet werden können, müssen Ausgangsmaterialien zur Verfügung stehen, die dem Polymer gute elektrische, chemische, mechanische und thermische Eigenschaften verleihen.

Monomere für die Herstellung von gut löslichen Polybenzoxazolvorstufen werden beispielsweise in der US 4 525 539 oder der EP 317 942 beschrieben.

Beispielsweise betrifft die EP 1 132 374 A2 Bis-o-aminophenole und o-Aminophenolcarbonsäuren folgender Struktur: 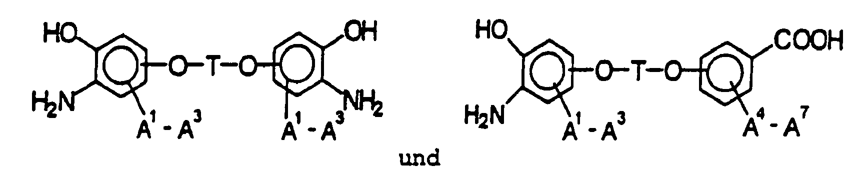 wobei A¹ bis A⁷ unabhängig voneinander H, F, CH₃, CF₃, OCH₃ oder OCF₃ sind und T einen aromatischen oder heterozyklischen Rest darstellt.

Ferner beschreibt die EP 0 906 903 A2 Bis-o-aminophenole und Bis-o-aminothiophenole folgender Struktur: 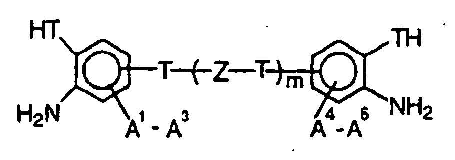 wobei A¹ bis A⁶ unabhängig voneinander sind: H, F, CH₃, CF₃, OCH₃, OCF₃, CH₂CH₃, CF₂CF₃, OCH₂CH₃ oder OCF₂CF₃, mindestens einer der Reste A¹ bis A⁶ F oder eine F-haltiger Gruppe sein muss; T O oder S ist und m 0 oder 1 ist und Z ein aromatischer oder heterocyclischer Rest ist.

Durch die ständig wachsenden Anforderungen an die Leistungsfähigkeit der Mikrochips und die damit einhergehende Miniaturisierung der Halbleiterbauelemente ist jedoch eine ständige Weiterentwicklung der Polymermaterialien erforderlich, um auch bei abnehmenden Abmessungen der Bauelemente die für die Polymere geforderten mechanischen, elektrischen und chemischen Eigenschaften erfüllen zu können. Dies erfordert wiederum eine Fortentwicklung der zur Verfügung stehenden Monomeren für die Herstellung von Polybenzoxazolen bzw. deren löslichen Vorstufen.

Aufgabe der Erfindung ist es daher, neue Ausgangsmaterialien zur Verfügung zu stellen, welche die Herstellung von isolierenden Polymeren ermöglichen, welche eine niedrige Dielektrizitätskonstante sowie eine hohe Temperatur und Chemikalienstabilität aufweisen.

Die Aufgabe wird gelöst mit Bis-o-aminophenolen der Formel I 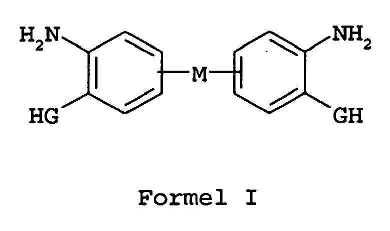 wobei bedeutet:
- G:: Sauerstoff oder Schwefel;
M: 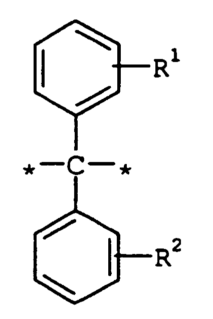 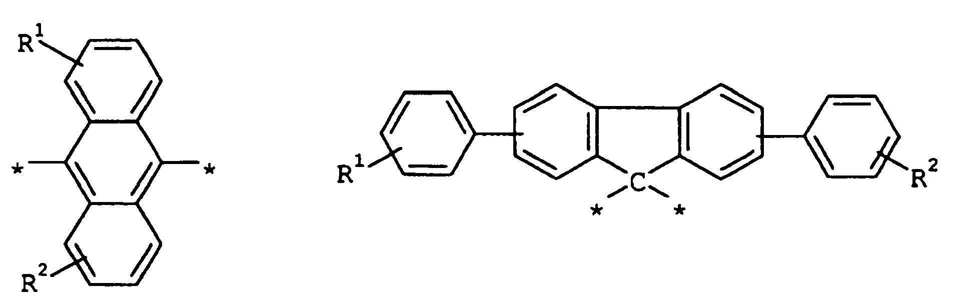 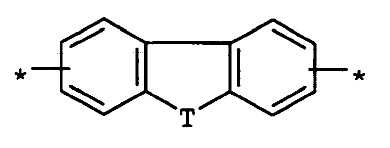 R¹, R², jeweils unabhängig: 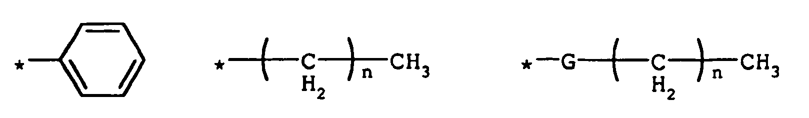 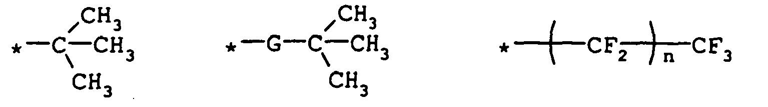 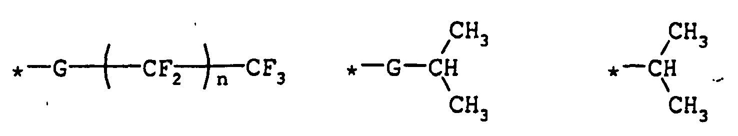 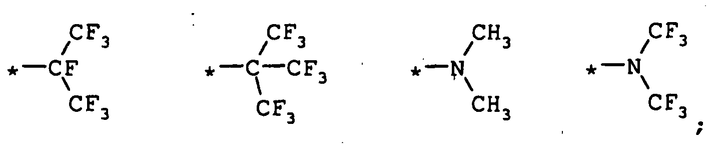 T: 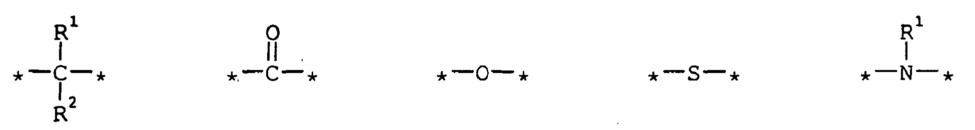
- n:: 0 bis 5.

Die erfindungsgemäßen Bis-o-aminophenole der Formel I eignen sich für die Herstellung von Poly-o-hydroxyamiden, aus denen polymere Dielektrika erhalten werden können, die eine Dielektrizitätskonstante von k ≦ 2,7 aufweisen. Diese Polymeren eignen sich sehr gut zum Füllen von engen Gräben. Nach der Cyclisierung des Poly-o-hydroxyamids werden Polybenzoxazole erhalten, die eine hohe Beständigkeit gegenüber Prozesschemikalien aufweisen, wie Lösungsmitteln, Strippern, Basen, Säuren oder aggressiven Gasen. Diese Polymeren eignen sich auch hervorragend für die Kupfer-Damascene-Technik. Während des Schleifprozesses, in dem überschüssiges Kupfer abgetragen wird, treten keine nachteiligen Effekte, wie Ablösungen, Riss- oder Blasenbildungen auf. Die Haftung solcher Dielektrika an den für die Chiptechnologie relevanten Oberflächen, wie Silizium, Siliziumcarbid, Siliziumcarbonitrid, Siliziumnitrid, Siliziumoxid, Titan, Tantal, Titannitrid, Tantalnitrid oder Siliziumoxynitrid ist sehr gut. Die aus den erfindungsgemäßen Bis-o-aminophenolen hergestellten Polymeren sind in vielen organischen Lösungsmitteln sehr gut löslich. Geeignete Lösungsmittel sind zum Beispiel Aceton, Cyclohexanon, Diethylenmono- bzw. -diethylether, N-Methylpyrrolidon, γ-Butyrolacton, Ethyllactat, Methoxymethylacetat, Tetrahydrofuran oder Essigsäureethylester. Sie lassen sich durch Schleuder-, Sprüh- oder Tauchtechniken gut verarbeiten und ergeben Filme von sehr guter Qualität. Die von den erfindungsgemäßen Bis-o-aminophenolen abgeleiteten Polybenzoxazole haben außerdem eine sehr hohe Temperaturstabilität.

Besonders bevorzugt sind Bis-o-aminophenole der Formel II 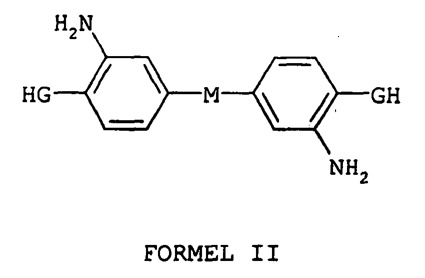 wobei M und G die oben angegebene Bedeutung aufweisen. Die Bis-o-aminophenole der Formel II lassen sich in hohen Ausbeuten und hoher Isomerenreinheit darstellen, was die Reinigung der Produkte wesentlich vereinfacht. Die Bis-o-aminophenole der Formel II können daher kostengünstig zur Verfügung gestellt werden.

Die erfindungsgemäßen Bis-o-aminophenole unterteilen sich in Bis-o-aminothiophenole (G = S) und Bis-o-aminophenole (G = O). Der Einfachheit halber werden beide Verbindungsklassen unter der Bezeichnung "Bis-o-aminophenole" zusammengefasst. Obwohl sich auch mit Bis-o-aminothiophenolen Polymere mit vorteilhaften Eigenschaften herstellen lassen, haben die Bis-o-aminophenole (G = O) die größere Bedeutung, da sie sich wegen ihrer höheren Oxidationsbeständigkeit leichter verarbeiten lassen. Unter den Bis-o-aminophenolen der Formel I sind daher die Verbindungen bevorzugt, in welchen G für ein Sauerstoffatom steht.

Um für eine industrielle Anwendung geeignet zu sein, ist es wesentlich, dass die Bis-o-aminophenole der Formel I einfach und kostengünstig zugänglich sind. Gegenstand der Erfindung ist daher auch ein Verfahren zur Herstellung von Bis-o-aminophenolen der Formel I, wobei ein Diol der Formel III 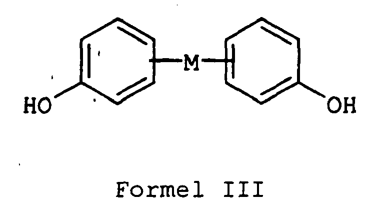 in welchem M die in Anspruch 1 angegebene Bedeutung aufweist, mit einem Nitrosieragens zur Nitrosoverbindung nitrosiert wird und die Nitrosoverbindung anschließend zum Bis-o-aminophenol der Formel I reduziert wird.

Für die Herstellung der Bis-o-aminophenole der Formel I wird das Diol der Formel III zunächst in einem geeigneten inerten Lösungsmittel bei Raumtemperatur gelöst. Geeignete Lösungsmittel sind beispielsweise Alkohole, wie Ethanol, Isopropanol oder Butanol, Acetonitril, Ester, wie Ethylacetat oder Propylacetat, Ketone, wie Aceton, Methylethylketon oder Diethylketon, oder chlorierte Kohlenwasserstoffe wie Chloroform, Dichlormethan oder Methylenchlorid. Die Lösung enthält dabei vorzugsweise 5 bis 30 Gew.-% des als Edukt verwendeten Diols der Formel III. Anschließend wird ein geeignetes Nitrosierungsagens zugegeben und die erhaltene Mischung bei Temperaturen von vorzugsweise -15 bis 40°C, insbesondere 5 bis 15°C gerührt, bis ein annähernd vollständiger Umsatz des Edukts stattgefunden hat. Geeignete Reaktionszeiten liegen im Allgemeinen im Bereich von 1 - 10 Stunden, insbesondere 2 - 4 Stunden. Um die entstandene Nitrosoverbindung abzutrennen, wird zunächst das Lösungsmittel verdampft, vorzugsweise unter vermindertem Druck. Anschließend kann die Nitrosoverbindung durch Umkristallisation aus einem geeigneten Lösungsmittel oder durch säulenchromatografische Trennung mit einem geeigneten Eluenten gereinigt werden.

Der Vorteil des erfindungsgemäßen Verfahrens ist zum einen, dass das als Edukt verwendete Diol der Formel III eine ausreichende Reaktivität aufweist, um innerhalb von für eine industrielle Anwendung interessanten Zeiträumen eine weitgehend vollständige Umsetzung der Dihydroxyverbindung zu erreichen. Ferner müssen die Hydroxygruppen der Dihydroxyverbindung der Formel III nicht durch eine Schutzgruppe geschützt werden, so dass Arbeitsschritte zur Einführung bzw. Abspaltung einer Schutzgruppe entfallen. Steht die Hydroxygruppe in paraStellung zur Gruppe M, wird die Nitrosogruppe selektiv in ortho-Stellung zur Hydroxygruppe in den Phenylring eingeführt, so dass bei der nachfolgenden Reinigung keine Abtrennung von unerwünschten Isomeren erforderlich ist. Auch dies trägt zur kostengünstigen Herstellung der Bis-ortho-aminophenole der Formel I bei.

Für die Nitrosierung können alle üblichen Nitrosierungsagentien verwendet werden. Geeignet sind beispielsweise Isoamylnitrit, Alkylnitrite sowie ein Gemisch aus Natriumnitrit und konzentrierter Schwefelsäure.

Die gereinigte Bis-o-nitrosoverbindung wird anschließend zur Bis-o-aminoverbindung reduziert. Dazu wird die Nitrosoverbindung zunächst in einem geeigneten Lösungsmittel gelöst. Geeignet sind beispielsweise Ether, wie Tetrahydrofuran oder Dioxan. Die Reduktion zur Aminogruppe erfolgt vorzugsweise mit Wasserstoff unter Katalyse durch einen geeigneten Katalysator. Zur Beschleunigung der Reaktion erfolgt die Hydrierung vorteilhaft bei erhöhtem Wasserstoffdruck. Ein geeigneter Katalysator ist beispielsweise Palladium auf Aktivkohle.

Die Bis-o-aminophenole der Formel I lassen sich auch über die entsprechenden Nitroverbindungen herstellen. Gegenstand der Erfindung ist daher auch ein Verfahren zur Herstellung von Bis-o-aminophenolen der Formel I, wobei ein Diol der Formel IV 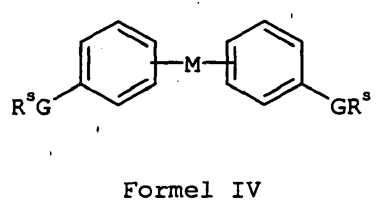 in welchem M und G die in Anspruch 1 angegebene Bedeutung aufweisen und R^{s} eine Schutzgruppe ist, mit einem Nitrieragens zur Nitroverbindung nitriert wird und die Nitroverbindung anschließend zum Bis-o-aminophenol der Formel I reduziert wird.

Die Nitrierung des geschützten Diols der Formel IV erfolgt mit üblichen Nitrierreagentien, beispielsweise Salpetersäure, Salpetersäure-Schwefelsäuregemischen, Distickstoffpentoxid oder Acetylnitrat. Bei dieser Reaktion ist allerdings erforderlich, dass die Hydroxygruppen des Edukts durch entsprechende Schutzgruppen R^{s} geschützt sind. Nach einer Reinigung der Bis-nitroverbindung wird die Nitrogruppe zur Aminogruppe reduziert. Dazu wird die Bis-nitroverbindung in einem geeigneten Lösungsmittel gelöst, beispielsweise Tetrahydrofuran, mit einem Katalysator versetzt, beispielsweise Palladium auf Aktivkohle, und in einem Autoklaven mit Wasserstoff hydriert.

Die Schutzgruppe R^{s} wird geeignet so ausgewählt, dass sie bei der Reduktion der Nitrogruppe zur Aminogruppe reduktiv abgespalten wird. Die Abspaltung der Schutzgruppe R^{s} erfordert in diesem Fall keinen zusätzlichen Produktionsschritt. Besonders geeignet wird als Schutzgruppe R^{s} eine Benzylgruppe verwendet.

Die Bis-o-aminophenole der Formel I lassen sich mit Dicarbonsäuren bzw. deren aktivierten Derivaten zu den gewünschten Poly-o-hydroxyamiden umsetzen. Dazu werden die Bis-o-aminophenole der Formel I mit einer Dicarbonsäure oder einem aktivierten Dicarbonsäurederivat der Formel V 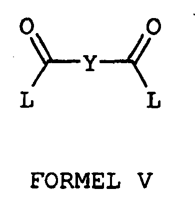 umgesetzt, wobei L für eine Hydroxygruppe oder eine aktivierende Gruppe steht und Y ein an sich beliebiger zweiwertiger Kohlenwasserstoffrest ist. Als aktivierende Gruppe für die Dicarbonsäurederivate der Formel V können zum Beispiel Säurechloride oder aktivierte Ester, zum Beispiel Sulfonsäureester verwendet werden. Die Umsetzung der Bis-o-aminophenole der Formel I und der Dicarbonsäuren der Formel V kann jedoch auch in Gegenwart einer die Carbonsäure aktivierenden Verbindung, wie zum Beispiel Carbonyldiimidazol oder Dicyclohexylcarbodiimid erfolgen. Im Prinzip eignen sich alle Reagentien, die das bei der Reaktion entstandene Wasser an sich binden. Für die Herstellung der Poly-o-hydroxyamide werden die Bis-o-aminophenole der Formel I und die Dicarbonsäure bzw. gegebenenfalls das Dicarbonsäurederivat der Formel V in einem organischen Lösungsmittel bei -20 bis 150°C innerhalb von 5 bis 20 Stunden zur Reaktion gebracht. Bei Bedarf können die Endgruppen des Polymers mit einem geeigneten Reagenz blockiert werden. Das nach der Reaktion entstandene Poly-o-hydroxyamid wird durch Zutropfen der Reaktionslösung in ein Fällungsmittel gefällt, gewaschen und getrocknet. Geeignete Fällungsmittel sind Wasser, sowie Alkohole, wie Isopropanol, Butanol oder Ethanol. Es können auch Mischungen dieser Fällmittel verwendet werden. Geeignet kann das Fällungsmittel auch von 0,1 % bis zu 10 % Ammoniak enthalten. Das ausgefallene Polymer kann durch Filtration und Trocknung unmittelbar weiterverarbeitet werden und zum Beispiel für den Auftrag auf ein Halbleitersubstrat in einem der weiter oben genannten Lösungsmittel gelöst werden.

Die Polymerisation zum Poly-o-hydroxyamid kann in Gegenwart einer Base durchgeführt werden, um frei werdende Säure abzufangen. Geeignete basische Säurefänger sind beispielsweise Pyridin, Triethyl-amin, Diazabicyclooctan oder Polyvinylpyridin. Es können aber auch andere basische Säurefänger verwendet werden. Insbesondere bevorzugt werden Verbindungen, die im für die Synthese verwendeten Lösungsmittel, zum Beispiel N-Methylpyrrolidon und im Fällungsmittel, zum Beispiel Wasser oder Wasser-Alkohol-Mischungen gut löslich sind, oder solche, die im Lösungsmittel vollkommen unlöslich sind, wie zum Beispiel vernetztes Polyvinylpyridin. Die Säurefänger lassen sich dann bei der Aufarbeitung des Reaktionsprodukts leicht vom entstandenen Poly-o-hydroxyamid abtrennen.

Besonders geeignete Lösungsmittel für die Polymersynthese sind γ-Butyrolacton, Tetrahydrofuran, N-Methylpyrrolidon und Dimethylacetamid. An sich kann jedoch jedes Lösungsmittel verwendet werden, in dem die Ausgangskomponenten gut löslich sind.

Die auf diese Weise dargestellten Poly-o-hydroxyamide lassen sich durch Erwärmen unter Cyclisierung nach dem oben erläuterten Mechanismus in die gewünschten Polybenzoxazole überführen. Diese eignen sich wegen ihrer guten elektrischen, mechanischen und chemischen Eigenschaften sehr gut für eine Verwendung in der Mikroelektronik.

Die Erfindung wird anhand von Beispielen näher erläutert.

### Beispiel 1: Synthese von 3,3' - Diamino-4,4' dihydroxytetraphenylmethan

### Syntheseweg:

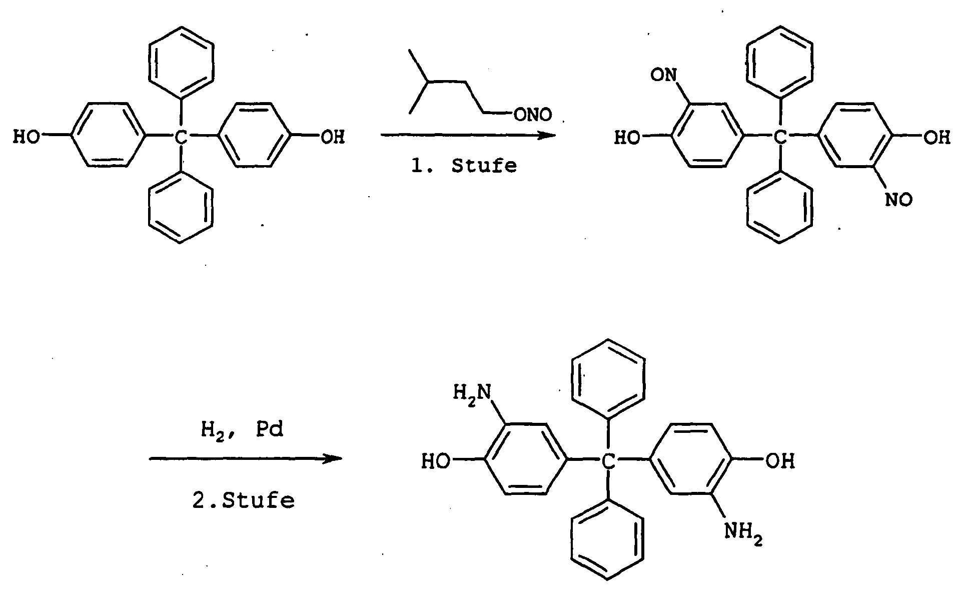

### Stufe 1: Nitrosierung

20,07 g (0,057 mol) 4,4'-Dihydroxytetraphenylmethan werden in 100 ml Eisessig gelöst und bei RT tropfenweise mit 20 ml Isoamylnitrit versetzt. Der Verlauf der Umsetzung wird dünnschichtchromatographisch verfolgt, bis kein Ausgangsstoff mehr nachweisbar ist. Es wird 100 - 200 ml Wasser zugesetzt und vom ausfallenden Feststoff abfiltriert Dieser wird mit Methanol verrieben und mehre Stunden Stehen gelassen. Nach Absaugen und Trocknen wird das Produkt in heißem Toluol aufgenommen und fraktioniert zur Kristallisation gebracht.
Ausbeute: 21,27 g (91 % d. Theorie)

### Stufe 2: Hydrierung und Isolierung als Hydrochlorid

Die Hydrierung erfolgt entsprechend bekannten Verfahren zur Hydrierung von Nitroverbindungen, wie z.B. in EP 905121 Beispiel 8 beschrieben.

20,5 g (0,05 mol) 4,4'-Dihydroxy-3,3'-dinitrosotetraphenylmethan werden in 250 ml Tetrahydrofuran (THF) aufgelöst und unter Schutzgas mit 2,00 g Pd-C 5% versetzt. Die Suspension wird in einen zuvor ausgeheizten Hydrierreaktor unter Ar-Schutzgas eingefüllt und bei Raumtemperatur für 24 h und 2 bar H₂-Druck hydriert. Nach 24 h Hydrierzeit wird die Suspension unter Schutzgas in 150 ml Ethanol p.A. überführt. Die Mischung wird unter Rühren mit 10 ml konz. HCl versetzt und bei vollständig gelöstem Produkt dreimal über ein Büchnertrichter abfiltriert, um den Pd-Katalysator zu entfernen. Die so erhaltene Lösung wird bei 70 °C und 300 mbar eingeengt bis ca. 20 ml Ethanol zurückbleiben und unter schnellem Rühren in eine Lösung aus 700 ml Diethylether und 30 ml Aceton (Selectipur) gegeben. Die Suspension wird für 24 h bei -18 °C gelagert. Anschließend wird der Feststoff abgesaugt und getrocknet.
Ausbeute: 21,39 g (94% d. Theorie)

### Beispiel 2: Synthese von 9,10-Bis-(3-amino-4-hydroxyphenyl)-anthracen

### Syntheseweg:

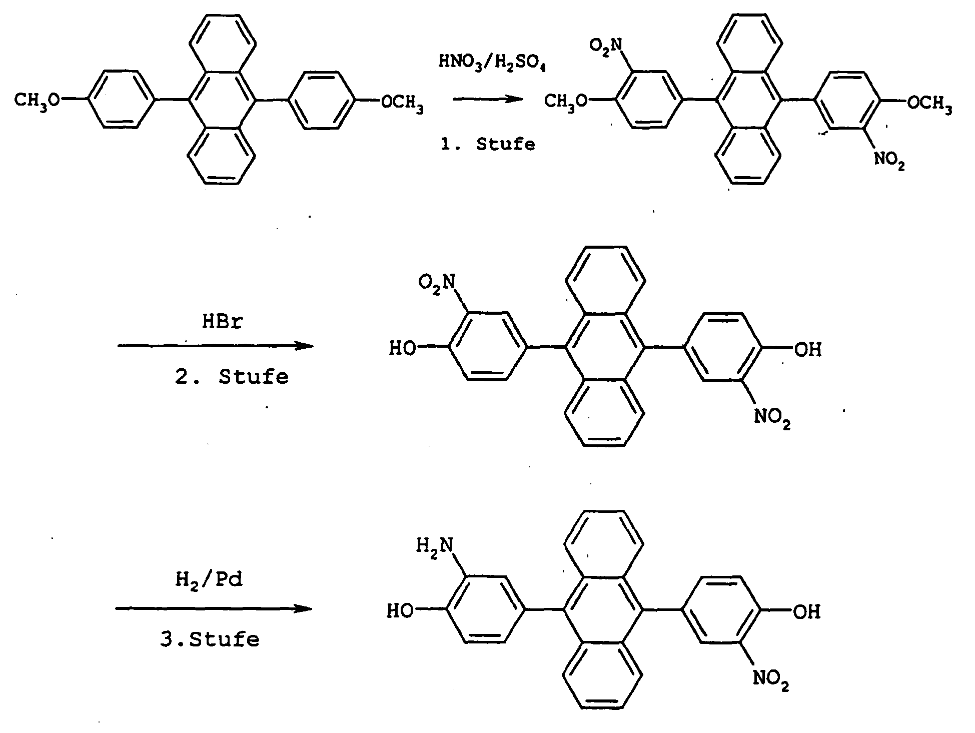

### Stufe 1: 9,10-Bis-(4-methoxy-3-nitrophenyl)-anthracen

23,43 g (60 mmol) 9,10-Bis-(4-methoxy)-anthracen werden in 250 ml Acetanhydrid vorgelegt und gelöst. Anschließend wird eine Lösung aus 18,7 ml (0,184 mol) Salpetersäure (62 %-ig) in 100 ml Acetanhydrid innerhalb 30 min zur 0 °C kalten Lösung zugetropft. Bei gleicher Temperatur wird für 4 h gerührt und der ausgefallene Feststoff auf einer Fritte abgesaugt. Nach beendeter Reaktion wird die Mischung vorsichtig in 500 ml Eiswasser gegossen und gut durchgerührt. Das Produkt wird abgesaugt, gründlich mit Wasser gewaschen und umkristallisiert. Das Rohprodukt wird dazu bei Raumtemperatur in Toluol gelöst (2 ml/g), auf 90 °C erhitzt und mit (4 ml/g) Petrolether versetzt, bis eine Kristallisation einsetzt. Die Mischung wird anschließend langsam auf RT abgekühlt. Die Suspension wird für 4 h im Kühlschrank bei -18 °C gelagert und anschließend abfiltriert. Produkt wird für 24 h bei 200 mbar und 55 °C getrocknet.
Ausbeute: 21,9g (76% d. Theorie)

### Stufe 2: 9,10-Bis-(4-hydroxy-3-nitrophenyl)-anthracen

19,2 g (0,04 mol) 9,10-Bis-(4-methoxy-3-nitrophenyl)-anthracen werden in einem Gemisch aus 200 ml Butanon und 200 ml konz. HBr 24 h unter Rühren im Rückfluss erhitzt. Die Suspension wird nach beendeter Reaktion am Rotationsverdampfer auf die Hälfte ihres Volumens eingeengt und das Produkt abgesaugt. Abschließend wird aus Toluol umkristallisiert.
Ausbeute: 16,64g (92 % d. Theorie)

### Stufe 3: 9,10-Bis-(3-amino-4-hydroxyphenyl)-anthracen

Die Hydrierung erfolgt entsprechend den bekannten Verfahren zur Hydrierung von Nitro-verbindungen, wie z.B. in EP 905121 Beispiel 8 beschrieben.

13,56 g (30 mmol) 9,10-Bis-(4-hydroxy-3-nitrophenyl)-anthracen werden in 300 ml THF aufgelöst und unter Schutzgas mit 3,00 g Pd-C 5 % versetzt. Die Suspension wird in einen zuvor ausgeheizten Hydrierreaktor unter Ar-Schutzgas eingefüllt und bei Raumtemperatur für 24 h und 2 bar H₂-Druck hydriert. Nach 24 h Hydrierzeit wird die Suspension unter Schutzgas in 100 ml Ethanol p.A. überführt. Die Mischung wird unter Rühren mit 10 ml konz. HCl versetzt und bei vollständig gelöstem Produkt 3x über ein Büchnertrichter abfiltriert, um den Pd-Katalysator zu entfernen. Die so erhaltene Lösung wird bei 70 °C und 300 mbar eingeengt bis ca. 20 ml Ethanol zurückbleiben und unter schnellem Rühren in eine Lösung aus 500 ml Diethylether und 20 ml Aceton (Selectipur) gegeben. Hierbei fällt das Produkt in Form dunkelvioletter Kristalle aus. Die Suspension wird für 24 h bei -18 °C gelagert. Anschließend wird der Feststoff abgesaugt und getrocknet.

Ausbeute: 12,37 g (89 % d. Theorie)

## Patentansprüche

1. Bis-o-aminophenole der Formel I 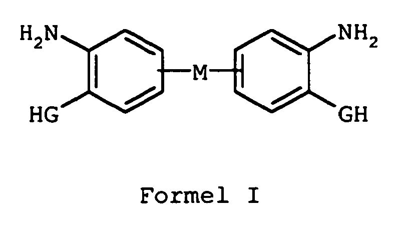 wobei bedeutet:
G: Sauerstoff oder Schwefel;
M: 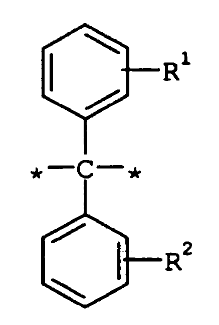 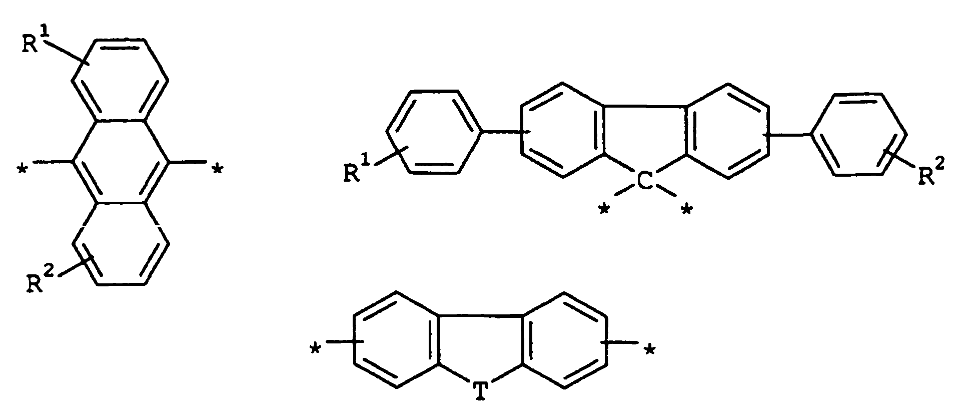 R¹, R², jeweils unabhängig: 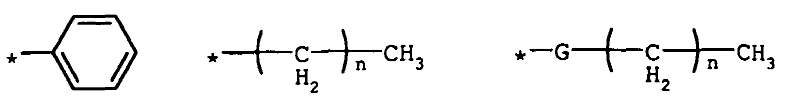 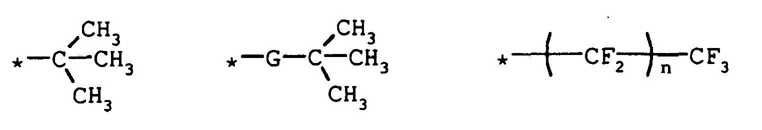 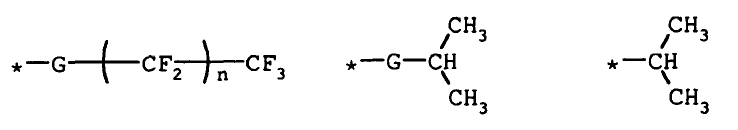 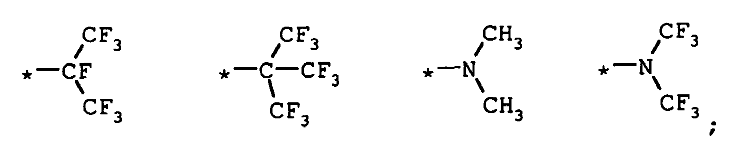 T: 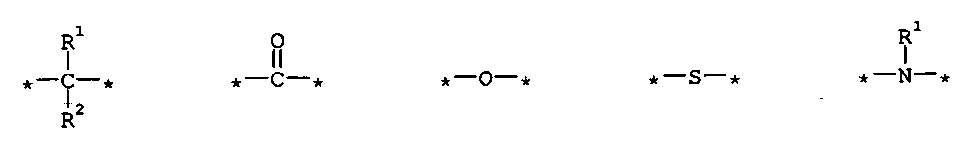
n: 0 bis 5.

2. Bis-o-aminophenole nach Anspruch 1 der Formel II 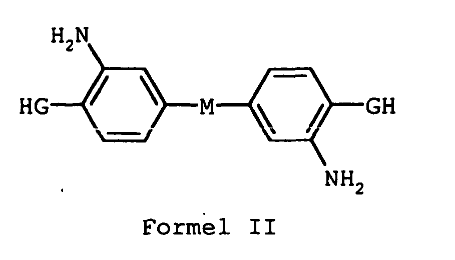 wobei M und G die in Anspruch 1 angegebene Bedeutung aufweisen.

3. Bis-o-aminophenole nach Anspruch 1 oder 2, wobei G ein Sauerstoffatom bedeutet.

4. Verfahren zur Herstellung von Bis-o-aminophenolen der Formel I, wobei ein Diol der Formel III 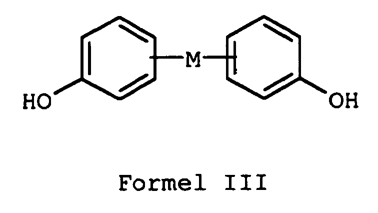 in welchem M die in Anspruch 1 angegebene Bedeutung aufweist, mit einem Nitrosieragens zur Nitrosoverbindung nitrosiert wird und die Nitrosoverbindung anschließend zum Bis-o-aminophenol der Formel I reduziert wird.

5. Verfahren nach Anspruch 4, wobei die Nitrosoverbindung unter Wirkung eines Katalysators mit Wasserstoffgas reduziert wird.

6. Verfahren nach Anspruch 5, wobei der Katalysator Palladium auf Aktivkohle ist.

7. Verfahren nach einem der Ansprüche 4 bis 6, wobei das Nitrosieragens ausgewählt ist aus der Gruppe, die gebildet ist aus Isoamylnitrit, Alkylnitriten sowie einem Gemisch aus Natriumnitrit und konzentrierter Schwefelsäure.

8. Verfahren zur Herstellung von Bis-o-aminophenolen der Formel I, wobei ein Diol der Formel IV 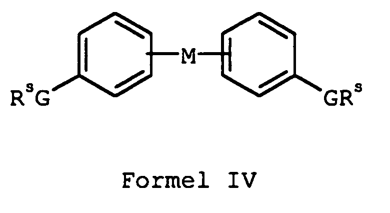 in welchem M und G die in Anspruch 1 angegebene Bedeutung aufweisen und R^{s} eine Schutzgruppe ist, mit einem Nitrieragens zur Nitroverbindung nitriert wird und die Nitroverbindung anschließend zum Bis-o-aminophenol der Formel I reduziert wird.

9. Verfahren nach Anspruch 8, wobei die Schutzgruppe R^{s} eine reduktiv abspaltbare Gruppe ist.

10. Verfahren nach einem der Ansprüche 8 oder 9, wobei die Schutzgruppe R^{s} eine Benzylgruppe ist.

## Claims

1. Bis-o-aminophenols of the formula I 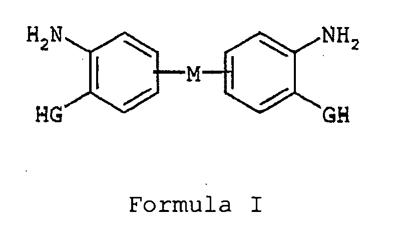 in which
G is oxygen or sulfur;
M is 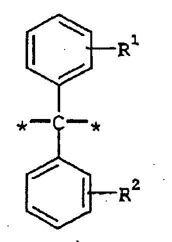 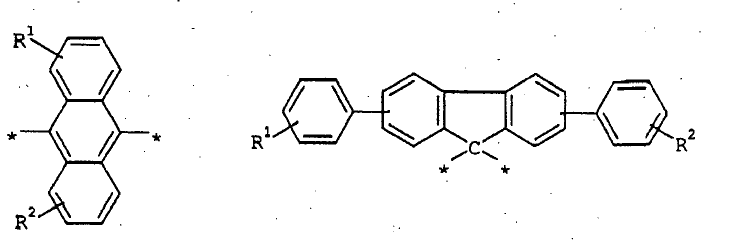 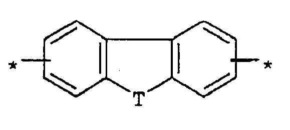
R¹, R², in each case independently, are 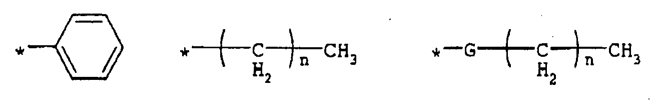 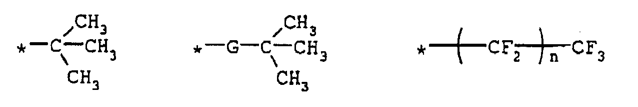 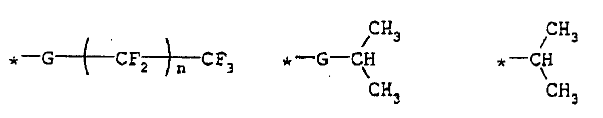 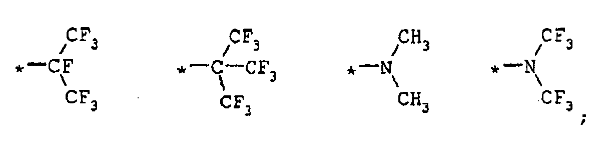
T is 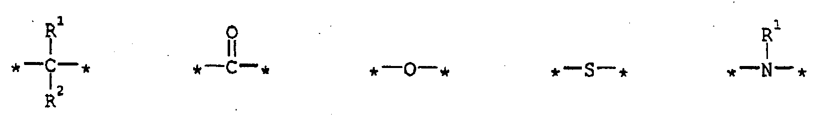
n is from 0 to 5.

2. Bis-o-aminophenols according to Claim 1, of the formula II 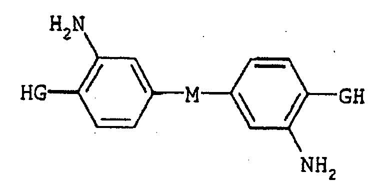 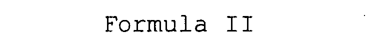 in which M and G have the meaning stated in Claim 1.

3. Bis-o-aminophenols according to Claim 1 or 2, wherein G is an oxygen atom.

4. Process for the preparation of bis-o-aminophenols of the formula I, wherein a diol of the formula III 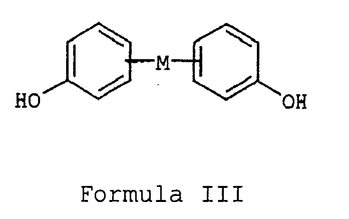 in which M has the meaning stated in Claim 1, is nitrosated with a nitrosating agent to give the nitroso compound, and the nitroso compound is then reduced to the bis-o-aminophenol of the formula I.

5. Process according to Claim 4, wherein the nitroso compound is reduced with hydrogen gas under the action of a catalyst.

6. Process according to Claim 5, wherein the catalyst is palladium on active carbon.

7. Process according to any of Claims 4 to 6, wherein the nitrosating agent is selected from the group consisting of isoamyl nitrite, alkyl nitrites and a mixture of sodium nitrite and concentrated sulfuric acid.

8. Process for the preparation of bis-o-aminophenols of the formula I, wherein a diol of the formula IV 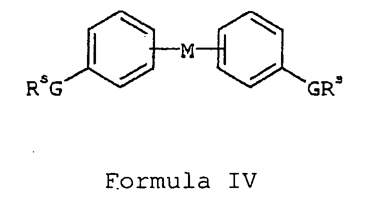 in which M and G have the meaning stated in Claim 1 and R^{s} is a protective group, is nitrated with a nitrating agent to give the nitro compound, and the nitro compound is then reduced to give the bis-o-aminophenol of the formula I.

9. Process according to Claim 8, wherein the protective group R^{s} is a group capable of undergoing reductive elimination.

10. Process according to either of Claims 8 and 9, wherein the protective group R^{s} is a benzyl group.

## Revendications

1. Bis-o-aminophénol de Formule I 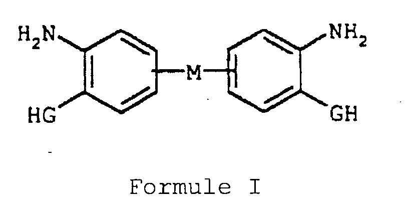 avec les significations suivantes :
G : oxygène ou soufre ;
M : 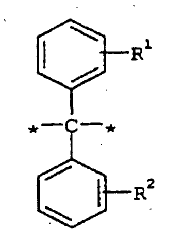 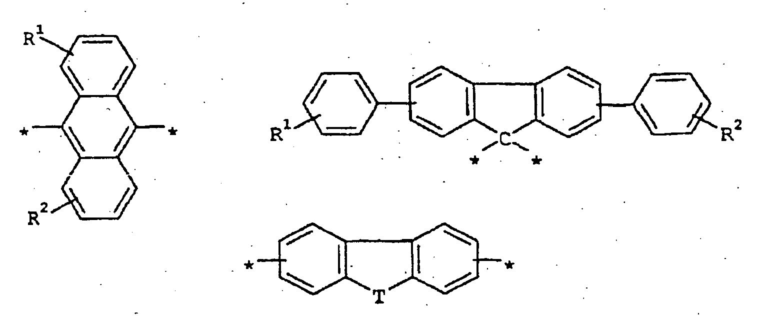 R¹, R², respectivement indépendamment : 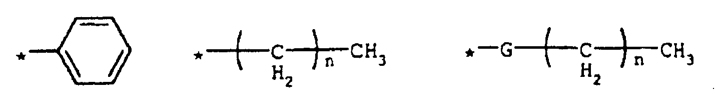 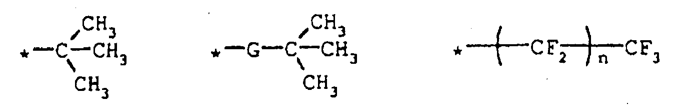 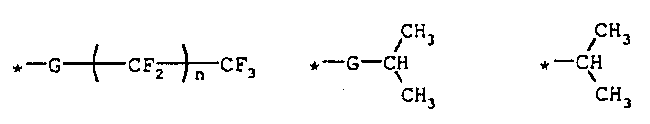 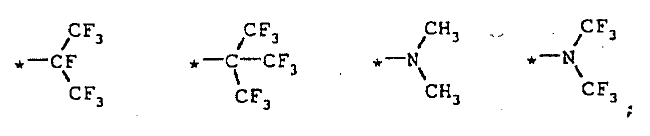 T: 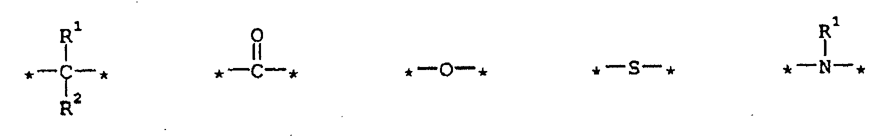
n : 0 à 5.

2. Bis-o-aminophénol suivant la revendication 1 de Formule II 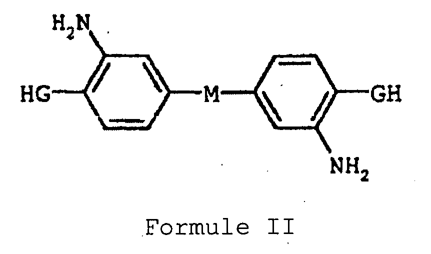 dans laquelle M et G ont la signification indiquée à la revendication 1.

3. Bis-o-aminophénol suivant la revendication 1 ou 2, dans lequel G signifie un atome d'oxygène.

4. Procédé de préparation de bis-o-aminophénol de Formule I, dans lequel on nitrose en un composé nitroso un diol de Formule III 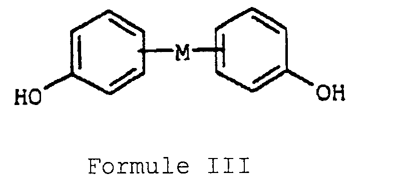 dans laquelle M a la signification indiquée à la revendication 1, par un réactif de nitrosation et on réduit ensuite le composé nitroso en le bis-o-aminophénol de Formule I.

5. Procédé suivant la revendication 4, dans lequel on réduit le composé nitroso par de l'hydrogène gazeux sous l'action d'un catalyseur.

6. Procédé suivant la revendication 5, dans lequel le catalyseur est du palladium sur charbon actif.

7. Procédé suivant l'une des revendications 4 à 6, dans lequel on choisit le réactif de nitrosation dans le groupe formé du nitrite d'isoamyle, de nitrite d'alcoyle, ainsi que d'un mélange de nitrite de sodium et d'acide sulfurique concentré.

8. Procédé de préparation du bis-o-aminophénol de Formule I, dans lequel on nitre en un composé nitro, par un agent de nitration, un diol de Formule IV 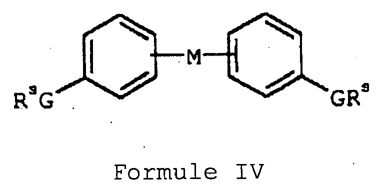 dans laquelle M et G ont la signification indiquée à la revendication 1 et R^{s} est un groupe de protection, et on réduit ensuite le composé nitro en le bis-o-aminophénol de Formule I.

9. Procédé suivant la revendication 8, dans lequel le groupe R^{s} de protection est un groupe labile par réduction.

10. Procédé suivant l'une des revendications 8 ou 9, dans lequel le R^{s} de protection est un groupe benzyle.
